# EUROPEAN PATENT SPECIFICATION

(11) **EP 1 556 517 B1**
(45) Date of publication and mention of the grant of the patent: **08.03.2006**
(21) Application number: 03788980.5
(22) Date of filing: 30.10.2003
(51) Int. Cl.: C12Q 1/68

(54) **IMPROVED METHODS FOR GENERATING MULTIPLE RNA COPIES**
VERBESSERTE METHODEN ZUR ERZEUGUNG EINER VIELZAHL VON RNA-KOPIEN
PROCEDES AMELIORES PERMETTANT DE GENERER DE MULTIPLES COPIES D'ARN

(30) Priority: 30.10.2002 EP 02447204; 17.01.2003 US 440688 P
(43) Date of publication of application: 27.07.2005
(73) Proprietor: PamGene B.V., 5211 RX Den Bosch (NL)
(72) Inventor: BOENDER, Piet, NL-6522 KJ Nijmegen (NL)
(74) Representative: De Clercq, Ann G. Y.
(86) International application number: PCT/EP2003/012058
(87) International publication number: WO 2004/044239

(56) References cited:
- EP-A- 0 721 988
- WO-A-99/43850
- LOGEL J ET AL: "SYNTHESIS OF CRNA PROBES FROM PCR-GENERATED DNA" BIOTECHNIQUES, EATON PUBLISHING, NATICK, US, vol. 13, no. 4, 1992, pages 604-606,608, XP002947579 ISSN: 0736-6205
- DEIMAN BIRGIT ET AL: "Characteristics and applications of nucleic acid sequence-based amplification (NASBA)." MOLECULAR BIOTECHNOLOGY, vol. 20, no. 2, February 2002 (2002-02), pages 163-179, XP009007141 ISSN: 1073-6085

## Description

### FIELD OF THE INVENTION

The present invention is directed to a novel method of efficiently synthesizing, in a non-specific manner, multiple copies of a target RNA. The present invention also relates to kits relating to the same and the use of these copies for determining gene expression patterns.

### BACKGROUND OF THE INVENTION

The detection and/or quantitation of specific nucleic acid sequences is an increasingly important technique for identifying and classifying micro organisms, diagnosing infectious diseases, detecting and characterizing genetic abnormalities, identifying genetic changes associated with cancer, studying genetic susceptibility to disease, and measuring response to various types of treatment. Such procedures have also found expanding uses in detecting and quantitating micro organisms in foodstuffs, environmental samples, seed stocks, and other types of material where the presence of specific micro organisms may need to be monitored. Other applications are found in the forensic sciences, anthropology, archaeology, and biology where measurement of the relatedness of nucleic acid sequences has been used to identify criminal suspects, resolve paternity disputes, construct genealogical and phylogenetic trees, and aid in classifying a variety of life forms.

Furthermore, in cells of higher organisms only some 15% of the genes present is expressed. Gene expression varies between different cell types and between different stages of development of a given cell and is crucial to all biological processes, such as aging, cell differentiation, and infectious or other disease states. Thus the identification of genes that are differentially expressed in cells under different conditions is of prime interest in cellular biology.

To be able to analyse the mRNA content derived from only a few cells a method is needed to amplify the mRNA present in the cell(s) under investigation. Much effort has already been put in methods to examine the mRNA population of a cell. This has led to the development of techniques to label nucleic acid material starting from the mRNA population of a cell aimed at the identification of genes that are differentially expressed in cells under various conditions.

A common method for detecting and quantitating expression of specific nucleic acid sequences is nucleic acid hybridisation, which is well known in the art. The sensitivity of nucleic acid hybridisation assays is limited primarily by the specific activity of the probe, the rate and extent of the hybridisation reaction, the performance of the method for separating hybridised and unhybridised probe, and the sensitivity with which the label can be detected. Researchers may need to detect and/or quantitate a specific gene sequence that is present as only a tiny fraction of all the sequences present in an organism's genetic material or in the messenger RNA population of a group of cells.

As a result of the interactions among the various components and component steps of this type of assay, there is almost always an inverse relationship between sensitivity and specificity. Thus, steps taken to increase the sensitivity of the assay (such as increasing the specific activity of the probe) may result in a higher percentage of false positive test results. The linkage between sensitivity and specificity has been a significant barrier to improving the sensitivity of hybridisation assays. One solution to this problem would be to specifically increase the amount of target sequence present using an amplification procedure. Amplification of a unique portion of the target sequence without requiring amplification of a significant portion of the information encoded in the remaining sequences of the sample could give an increase in sensitivity while at the same time not compromising specificity.

Most procedures to amplify nucleic acids relate to the generation of DNA.

For instance, a method for specifically amplifying nucleic acid sequences termed the "polymerase chain reaction" or "PCR" has been described by Mullis et al. (See for instance U.S. patents 4,683,195, 4,683,202 and 4,800,159 and Methods in Enzymology, Volume 155, 1987, pp. 335-350). The procedure uses repeated cycles of primer-dependent nucleic acid synthesis occurring simultaneously using each strand of a complementary sequence as a template. PCR is, however, not directly applicable to RNA. First, the target RNA has to be converted into cDNA by reverse transcriptase. Further, the requirement of repeated cycling of reaction temperature between several different and extreme temperatures is a disadvantage of the PCR procedure.

The PCR procedure has been coupled to RNA transcription by incorporating a promoter sequence into one of the primers used in the PCR reaction and then, after amplification by the PCR procedure for several cycles, using the doubte-stranded DNA as template for the transcription of single-stranded RNA. (See, e.g. Murakawa *et al.,* (1988) DNA 7:287-295).

Methods for chemically synthesizing relatively large amounts of DNA of a specified sequence *in vitro* are well known to those skilled in the art; production of DNA in this way is now commonplace. However, these procedures are time-consuming and cannot be easily used to synthesize oligonucleotides much greater in length than about 100 bases. Also, the entire base sequence of the DNA to be synthesized must be known. These methods require an expensive instrument capable of synthesizing only a single sequence at one time. Operation of this instrument requires considerable training and expertise. Methods for the chemical synthesis of RNA have been more difficult to develop.

Nucleic acids may be synthesized by techniques which involve cloning or insertion of specific nucleic acid sequences into the genetic material of micro organisms so that the inserted sequences are replicated when the organism replicates. If the sequences are inserted next to and downstream from a suitable promoter sequence, RNA copies of the sequence or protein products encoded by the sequence may be produced. Although cloning allows the production of virtually unlimited amounts of specific nucleic acid sequences, due to the number of manipulations involved it may not be suitable for use in diagnostic, environmental, or forensic testing. Use of cloning techniques requires considerable training and expertise. The cloning of a single sequence may consume several man-months of effort or more.

Relatively large amounts of certain RNAs may be made using a recombinant single-stranded-RNA molecule having a recognition sequence for the binding of an RNA-directed polymerase, such as Q beta replicase (see, e.g., U.S. Patent No. 4,786,600 to Kramer, *et al*.). A number of steps are required to insert the specific sequence into a DNA copy of the variant molecule, clone it into an expression vector, transcribe it into RNA and then replicate it with Q beta replicase.

Another manner to synthesize RNA is by application of primers containing bacteriophage promoters hybridising to an mRNA template. The primers hybridise to the 3' end of the template, after which a polymerase binds to the single stranded primer and starts synthesis (WO 93/22461). However, the polymerases work very inefficiently in this setting, if at all.

In screening differences in gene expression, such as by several versions of Differential Display Comparison, cDNA is made starting with a primer using the mRNA as a template. However, the enzyme that is used for this reaction (reverse transcriptase) is hampered in the cDNA synthesis by structures in the mRNA. As a result, these methods are selective for mRNAs with little or no structure. This negative effect is further enhanced if the synthesized cDNA is amplified further, for instance by PCR. Due to the aforementioned, it is common practice to use a large sample amount in these type of expression profiling analysis. Thus, this technical threshold does not allow the analysis of only a few cells isolated on a cell sorter or a few cells isolated via micro dissection from a glass slide after microscope identification and selection.

A method for the non-specific amplification of mRNA has been described in WO 99/43850, in which a primer is employed that binds to the poly-A-tail of mRNAs. Hence, this method is only applicable to amplify a pool of messenger RNAs with a poly-A-tail. In addition, the primer may bind close to the coding region but also at considerable distance thereof, due to the length of the poly-A-tail, and thereby introducing "void" sequence information.

A method for amplification of RNA has been described in EP-A-0 721 988, in which a chimeric primer is used, consisting of a DNA part as well as an RNA part. The RNA part is used for hybridising to the target RNA. The manufacture, and thus the use of a chimeric primer is complicated.

### BRIEF DESCRIPTION OF THE FIGURES

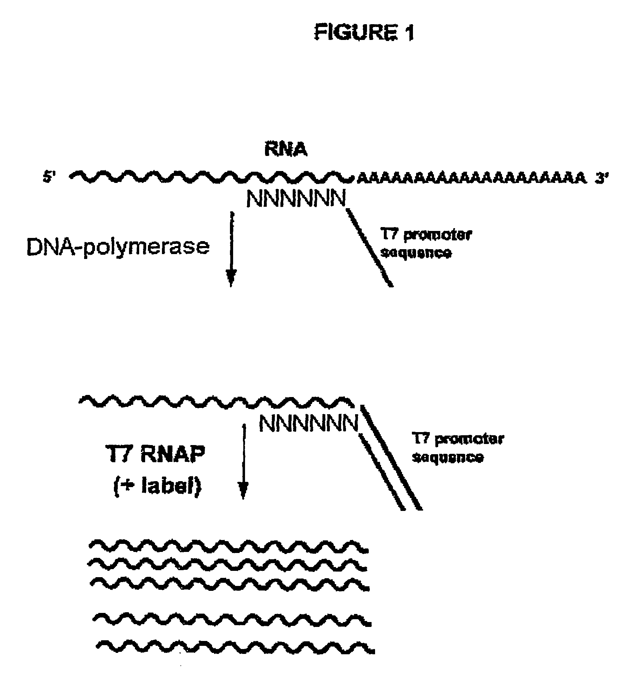

Figure 1: schematic representation of the amplification based on transcription. In this case, the target RNA (RNA) contains a poly-A tail, but it will be understood that any RNA can be the target RNA. The oligonucleotide primer comprising a random sequence is represented by the straight line attached to the random sequence "NNNNNN". In the figure, the enzyme with RNAse H activity has not been depicted, but might, for instance be inherent to the DNA polymerase, e.g. a reverse transcriptase. T7 RNAP is the T7 RNA polymerase.

### DETAILED DESCRIPTION OF THE INVENTION

The present invention is directed to a method and kit for the amplification of RNA target sequences. Such amplified target sequences are useful in assays for detecting gene expression differences between cell populations, the detection and/or quantitation of specific nucleic acid target sequences, for the labelling of specific nucleic acid target sequences, and for the production of large numbers of copies of RNA of specific target sequences for a variety of uses, to name a few.

In an embodiment, the invention provides a method for generating multiple RNA copies comprising the steps of:
(a) providing a sample comprising target RNA, wherein said sample is simultaneously contacted with:
   - an oligonucleotide comprising at its 5' side (possibly at its 5' end) a promoter sequence recognized by an RNA polymerase, wherein each oligonucleotide further comprises a target hybridising sequence, which is a random sequence; and
   - an enzyme having DNA polymerase activity;
   - an enzyme having RNase H activity;
   - an enzyme having RNA polymerase activity; and,
   - sufficient amounts of dNTPs and rNTPs; and,
(b) maintaining the resulting reaction mixture under the appropriate conditions for a sufficient amount of time for the enzymatic processes to take place.

Application of this method will lead to the formation of multiple RNA copies complementary to the target RNAs present in the reaction mixture. The method of the present invention does not need the production of cDNA intermediates as a basis for the amplification of the RNA. The RNA is synthesized by an RNA polymerase, directly from the target RNA template present in the material under investigation. The activity of the RNA polymerase is independent from any secondary structures present in the target RNA and thus there are no differences in the way the different target RNAs are amplified depending on structures in the target RNAs. The copies made represent the original target RNA population as present in the starting material. Dependent on the "target hybridising sequence" (see below) one target RNA species in a sample will be amplified, or more than one, or all target RNA species (if present).

The starting material is a sample comprising target RNA. The "target RNA" has a "target sequence" to be amplified, and may be either single-stranded or (partially) double-stranded and may include other sequences besides the target sequence which may not be amplified. The term "target sequence" refers to the particular nucleotide sequence of the target nucleic acid which is to be amplified. The "target sequence" includes the complexing sequences to which the oligonucleotides complex or hybridise during the processes of the present invention. Since the target nucleic acid is originally single-stranded, the term "target sequence" will also refer to the sequence complementary to the "target sequence" as present in the target nucleic acid.

The target RNA can be of eukaryotic, prokaryotic or viral origin, or a mixture thereof. The terms "of eukaryotic origin", "of prokaryotic origin" or "of viral origin" are all well known in the art.

The term "mixture" intends a sample comprising a combination of target RNA from eukaryotic, prokaryotic or viral origin, such as, for instance, a sample comprising target RNA from eukaryotic and prokaryotic origin or target RNA from eukaryotic and viral origin, target RNA from viral and prokaryotic origin. For instance, a sample from a mammalian patient, such as a human, may be infected or have been infected with a prokaryote, such as a bacteria, or a virus. Since the oligonucleotide comprising a target hybridising sequence, which may be a random sequence, will amplify all, or nearly all target RNAs in sample, by employing an applicable interrogating assay infectious prokaryotes and/or virus will be detected. Similarly, if oligonucleotides are used, which comprise an appropriately predetermined target hybridising sequence, i.e. specific for particular prokaryotes and/or virus, these prokaryotes and/or virus may be amplified, and detected by employing an applicable interrogating assay.

The target RNA can be derived from the group comprising total RNA, mRNA, cRNA, rRNA, tmRNA, asRNA, hnRNA or tRNA, including any combination thereof. In the case of eukaryotes, the term "hnRNA" relates to heterogeneous nuclear RNA, which are RNA polymerase II transcripts in the nucleus. The term "mRNA" relates to messenger RNA, without including or excluding precursor forms of the completely processed mRNA, such as mRNA which is in the process of capping, poly-A-addition and/or splicing. The term "rRNA" relates to ribosomal RNA, which is part of the ribosome, without including or excluding precursor forms of the completely processed rRNA, such as rRNA which is not spliced. The term "tRNA" relates to transfer RNA, without including or excluding aminoacyl-tRNA. The term "cRNA" relates to copy RNA, i.e. RNA synthesized from a template. The term "tmRNA" relates to bacterial RNA for its dual tRNA-like and mRNA-like nature (tmRNA is also known as 10Sa RNA or SsrA). The tmRNA engages in a trans-translation process, adding a C-terminal peptide tag to the unfinished protein on a stalled ribosome. The tmRNA-directed tag targets the unfinished protein for proteolysis. The term "asRNA" relates to anti-sense RNA, i.e. RNA synthesized from the minus strand, or RNA synthesized from other RNAs, including structural RNAs, such as rRNA and tRNA, and mRNA. The term "total RNA" relates to the complete RNA fraction of a cell, e.g. the total content of a cell, without including or excluding any of the aforementioned RNAs.

Discussions on nucleic acid synthesis are greatly simplified and clarified by adopting terms to name the two complementary strands of a nucleic acid duplex. Traditionally, the strand encoding the sequences used to produce proteins or structural RNAs was designated as the "plus" strand and its complement the "minus" strand. It is now known that in many cases, both strands are functional, and the assignment of the designation "plus" to one and "minus" to the other must then be arbitrary. Nevertheless, the terms are very useful for designating the sequence orientation of nucleic acids and will be employed herein for that purpose.

A "sample" or a "specimen" connotes a. collection of a small part of something intended as representative of the whole. The sample comprising target RNA relates to all starting materials derived from any source on which the method of the invention is applied. The term "sample" as used herein, may refer to a sample of tissue or fluid isolated from an individual, including but not limited to, for example, serum, plasma, lymph fluid, the external sections of the skin, respiratory intestinal, and genitourinary tracts, oocytes, tears, saliva, milk, blood cells, tumours, organs, gastric secretions, mucus, spinal cord fluid, external secretions, such as, for example, excrement, urine, sperm, and the like. As indicated above, the sample isolated from an individual may include target RNA from a different origin, e.g. of viral or prokaryotic origin. Samples are generally manipulated in order to isolate and/or characterise the target RNA. Also, the sample may be processed before applying it to the method of the invention. For example, target RNAs are generally isolated from a biological sample (cells, tissues, organs, etc.) and processed, using known in the art technology, such as isolation of, e.g. mRNA, cRNA, and the like. The sample comprising target RNA may be isolated from a tissue or cell of interest using any method known in the art. Similarly, and as mentioned above, the sample comprising target RNA may be derived from viral or prokaryotic origin.

The "oligonucleotide" of the invention is a stretch of nucleotides, which comprises a "target hybridising sequence" at the 3' side of the oligonucleotide and possibly at its 3' end, a promoter sequence 5' from the target hybridising sequence, such as at the 5' side, and possibly at its 5' end, and a transcription initiation region which is located between the target hybridising sequence and the promoter sequence of the promoter. Furthermore, the oligonucleotide is modified at its 3' end, by way of containing a modified nucleotide and a chimeric linkage between the nucleotides at the 3' end. The oligonucleotide may be produced naturally, synthetically or as a product of a restriction digest. The "target hybridising sequence" of the oligonucleotide is sufficiently complementary to a particular nucleotide sequence in the target RNA which complexes (by hydrogen bonding or hybridisation) with the target RNA to give an oligonucleotide/RNA complex. The "target hybridising sequence" may be a random or arbitrary sequence, such as, for instance, a stretch of 4, 5, 6, 7, 8, 9, or 10 randomly chosen nucleotides (normally characterised by "N"), or the "target hybridising sequence" may be a predetermined sequence. In case of a predetermined sequence, the sequence of the oligonucleotide is chosen such that the predetermined sequence will complex predominantly with the intended target RNA to be amplified. Complexing of the predetermined sequence with non-intended or accidental RNA will be less likely. In other words, the sequence of the oligonucleotide discriminates between hybridisation between intentional and accidental target RNA. For instance, the predetermined sequence may be chosen from the group comprising gene-specific sequences, mutation-specific sequences, poly-T sequences, genomic sequences, viral sequences, prokaryotic sequences, rRNA and the like. The person skilled in the art is aware that hybridisation is not only dependent on the complementary sequences, but also on the hybridisation conditions. The hybridisation conditions, such as hybridisation time, temperature, wash buffers used, etc. can be altered to optimise the efficient and specific binding of the target sequences. Suitable hybridisation conditions for various nucleic acid pairs are well known to those skilled in the art and reviewed in e.g. Sambrook et al., 1989 (in "Molecular Cloning: a, laboratory manual" 2nd edition; Cold Spring Harbor Laboratory Press, USA), which is herein specifically incorporated by reference. The terms "hybridise" and "hybridisation" refer to the formation of complexes between nucleotide sequences which are sufficiently complementary to form complexes via Watson-Crick base pairing. Where an oligonucleotide, i.e. the "target hybridising sequence", "hybridises" with target RNA (template) at the target sequence, such resulting complexes (or hybrids) are sufficiently stable to serve the priming function required by DNA polymerase, e.g. reverse transcriptase or Klenow pol I exo (-), to initiate DNA synthesis.

In an embodiment, the invention provides also a method for generating multiple RNA copies comprising the steps of:
(a) providing a sample comprising target RNA, wherein said sample is simultaneously contacted with:
   - a DNA oligonucleotide, comprising at its 5' side (possibly at its 5' end) a promoter sequence recognized by an RNA polymerase, wherein each oligonucleotide further comprises a target hybridising sequence, which is a predetermined sequence; and,
   - an enzyme having Klenow pol I exo (-) activity;
   - an enzyme having RNase H activity;
   - an enzyme having RNA polymerase activity; and,
   - sufficient amounts of dNTPs and rNTPs; and,
(b) maintaining the resulting reaction mixture under the appropriate conditions for a sufficient amount of time for the enzymatic processes to take place.

Upon hybridisation of the oligonucleotide to the target RNA, the target RNA is cut by an enzyme having RNase H activity, which generates a new 3' end of the target RNA. The newly generated 3' end of the RNA is extended by an enzyme having DNA polymerase activity, e.g. reverse transcriptase or Klenow pol I exo (-), on the oligonucleotide template to generate a double stranded promoter sequence. Hence, the enzyme having DNA polymerase activity synthesizes a complementary strand of the promoter sequence present in the oligonucleotide. To prevent any extension along the RNA template, the oligonucleotide is blocked at its 3' end. For instance, the reverse transcriptase will not be able to start extension of the blocked 3' end of the oligonucleotide and no cDNA is synthesized at this side. In case of DNA polymerases which cannot use the RNA template for synthesis, blocking of the oligonucleotide is not necessary, such as, for instance, enzymes having Klenow pol I exo (-) activity and T7 sequenase, which are both contemplated explicitly in the present invention. Klenow pol l exo (-) exhibits 5'=>3' polymerase activity of DNA polymerase I, *E.coli,* but lacks its 3'=>5' and 5'=>3' exonuclease activities (Derbyshire, *et al.,* Genetic and crystallographic studies of the 3',5'-exonucleolytic site of DNA polymerase l, Science, 240, 199-201, 1988). The oligonucleotide is made solely of DNA. In addition, the oligonucleotide, and preferentially the target hybridising sequence contains an extension-blocking modified nucleotide at its 3' terminal end and at least one chimeric linkage between nucleotides at the 3' end. (see below). The use of the oligonucleotide is depicted schematically in Figure 1.

The term "enzyme having DNA polymerase activity" relates to "DNA-dependent DNA polymerase" and "RNA-dependent DNA polymerase" or "reverse transcriptase". DNA polymerases require a template and synthesize a product whose sequence is complementary to that of the template. Most DNA-polymerases strongly prefer DNA templates (DNA-dependent DNA polymerases). The most frequently used DNA-dependent DNA polymerases are *E*. *coli* DNA polymerase I (holoenzyme and Klenow fragment) and DNA polymerases encoded by bacteriophages T4 and T7, modified bacteriophage T7 DNA polymerases (Sequenase™ and Sequenase version 2.0), and thermo stable DNA polymerases, such as Taq DNA polymerase and *Tht*. Although most DNA-dependent DNA polymerases strongly prefer DNA templates, they may also copy RNA, albeit at much lower efficiencies. Reverse transcriptase is an RNA-dependent DNA polymerase, i.e. an enzyme that synthesizes a complementary DNA copy from an RNA template. All known reverse transcriptases also have the ability to make a complementary DNA copy from a DNA template; thus, they may be regarded as both RNA- and DNA-dependent DNA polymerases. A primer is required to initiate synthesis with both RNA and DNA templates. In the present invention, the DNA polymerase extends the target RNA from the newly generated 3' end by the addition of covalently bonded bases linked at its 3' end which are complementary to the DNA template, i.e. the part of the oligonucleotide not complexed to the RNA, in the process of synthesis. The method of the invention contemplates reverse transcriptases such as, for instance, AMV-RT or MMLV-RT.

If the 3' terminus of the oligonucleotide would not be blocked and is complementary to the target nucleic acid, it may act as a primer and be extended by the DNA polymerase, using the target RNA as a template. In order to prevent or prohibit synthesis by the reverse transcriptase, the oligonucleotide is blocked at its 3' site. The 3' terminus of the oligonucleotide can be blocked in a variety of ways, including by a modification at its 3' terminal end. The modification at the 3' terminal end of the oligonucleotide can be accomplished by, for instance, having a 3'-terminal sequence non-complementary to the target RNA, or by having a biotin-group, or by having a modified nucleotide, such as a 3'-terminal dideoxynucleotide, Rp-NTP-α-S phosphorothioate nucleotide isomer and nucleotides comprising alkane-diol residues, cordycepins or amino-alkyls, or in other ways well known to those skilled in the art.

In order to facilitate the blocking function of the modified nucleotide at the 3' terminal end of the oligonucleotide, i.e. the prohibition of extension from this end, the present invention contemplates at least one chimeric linkage between nucleotides at the 3' end. For instance, most DNA polymerases have nuclease or exonuclease activity, such as for instance *E. coli* DNA polymerase I (Holoenzyme), which could degrade the oligonucleotide at its 3' end, including the removal of the blocking group, e.g. the modified nucleotide, the function of which is consequently destroyed. The term "chimeric linkage" relates to a linkage between the nucleotides which is different from the conventional 3'-5' phosphodiester linkage between the 5'-carbon of one nucleotide and the 3'-carbon of a second nucleotide, and which creates a sugar-phosphate-sugar backbone with the nucleotide bases sticking out. For instance, the chimeric linkage may contain at least one phosphorothioate linkage between nucleotides, or a PNA, LNA or GripNA backbone. In phosphorothioates, a single non-bridging oxygen atom bound to the phosphate atom is replaced by sulphur, as is well-known in the art. This chemical modification dramatically reduces the sensitivity to nuclease degradation. In PNA, the conventional backbone is replaced by a backbone which is made from repeating N-(2-aminoethyl)-glycine units linked by peptide bonds. The different bases (purines and pyrimidines) are linked to the PNA backbone by methylene carbonyl linkages. Unlike DNA or other DNA analogues, PNAs do not contain any pentose sugar moieties or phosphate groups.

PNAs with their peptide backbone bearing purine and pyrimidine bases are not a molecular species easily recognized by nucleases or proteases. They are thus resistant to the enzyme degradation. PNAs can be used in the same applications as traditional synthetic DNA or RNA. The term "PNA" includes "gripNA", which are negatively charged PNAs (Active Motif; Efimov VA, *et al.* (1998) NAR 26: 566-575; van der Laan, *et al.* (1996) Tetrahedron Lett. 37: 7857-7860; Efimov VA, *et al.* (1999) NAR 27: 4416-4426; all incorporated herein by reference). The term "LNA" or "Locked Nucleic Acid" has been coined to emphasize that the furanose ring conformation is restricted in LNA by a methylene linker that connects the 2'-O position to the 4'-C position. By convenience, all nucleic acids containing one or more LNA modifications are called LNA. LNA oligomers obey Watson-Crick base pairing rules and hybridise to complementary oligonucleotides. LNA provides vastly improved hybridisation performance when compared to DNA and other nucleic acid derivatives in a number of situations. The LNA modification has been shown to increase the biological stability of nucleic acids. Fully modified LNA oligonucleotides are resistant towards most nucleases tested.

In an embodiment, the invention provides also a method for generating multiple RNA copies comprising the steps of:
(a) providing a sample comprising target RNA; wherein said sample is simultaneously contacted with:
   - an oligonucleotide comprising at its 5' side (possibly at its 5' end) a promoter sequence recognized by an RNA polymerase, wherein each oligonucleotide further comprises:
      - a target hybridising sequence, wherein said hybridising sequence is a predetermined sequence,
      - a modified nucleotide at its 3' terminal end in such a way that extension there from is prohibited,
      - at least one chimeric linkage between nucleotides at the 3' end; and,
   - an enzyme having DNA polymerase activity;
   - an enzyme having RNase H activity;
   - an enzyme having RNA polymerase activity; and,
   - sufficient amounts of dNTPs and rNTPs; and,
(b) maintaining the resulting reaction mixture under the appropriate conditions for a sufficient amount of time for the enzymatic processes to take place.

A "promoter sequence" is a specific nucleic acid sequence that is recognized by a DNA-dependent RNA polymerase ("transcriptase" or "RNA polymerase") as a signal to bind to the nucleic acid and to begin the transcription of RNA in the 5'-> 3' direction at a position just downstream of the promoter. For binding, such transcriptases generally require DNA which is double-stranded in the portion comprising the promoter sequence and its complement; the template portion (sequence to be transcribed) need not be double-stranded. Individual DNA-dependent RNA polymerases recognize a variety of different promoter sequences which can vary markedly in their efficiency in promoting transcription. The different promoter sequences are well known in the art. The promoter sequences of the present invention contemplate the recognition sequence of the polymerase, as well as adjacent sequences 5' and/or 3' thereof, which may facilitate binding of the polymerase to its recognition sequence. When an RNA polymerase binds to a promoter sequence to initiate transcription, that promoter sequence is not part of the sequence transcribed. Thus, the RNA transcripts produced thereby will not include that sequence. The promoter may be the promoter for any suitable RNA polymerase. The present invention intends the use of an "enzyme having RNA polymerase activity", such as, for instance, the RNA polymerases from *E*. *coli* and bacteriophages T7, T3 and SP6, or any other suitable RNA polymerase. Accordingly, the promoter sequences that are recognised by the RNA polymerases from *E. coli* and bacteriophages T7, T3 and SP6, or any other suitable RNA polymerase are contemplated in the oligonucleotides of the invention. The processivity of, for example, the T7 RNA polymerase is very high, usually more than 250 nucleotides per second on a DNA template. This means that the amplification rate is determined by the number of initiation events per promoter, per time unit. Since the promoter is identical for each target RNA there is no selectivity in the amplification. By application of the RNA polymerase, new RNA copies of the original target RNA are made. During the transcription step, typically 10-1000, or 100-500 copies of each RNA are being made. Labels may be incorporated during the transcription step. The copies made are complementary to the target RNA, and in particular the target sequence.

A "template" is a nucleic acid molecule that is being copied by a nucleic acid polymerase. A template may be either single-stranded, double-stranded or partially double-stranded, depending on the polymerase. The synthesized copy is complementary to the template or to at least one strand of a double-stranded or partially double-stranded template. Both RNA and DNA are always synthesized in the 5' to 3' direction and the two strands of a nucleic acid duplex always are aligned so that the 5' ends of the two strands are at opposite ends of the duplex (and, by necessity, so then are the 3' ends).

An "RNase H" is an enzyme that degrades the RNA portion of an RNA:DNA duplex. RNase H's may be endonucleases or exonucleases. Most reverse transcriptase enzymes normally contain an RNase H activity in addition to their polymerase activity. However, other sources of the RNAse H are available without an associated polymerase activity. The RNase H may simply cut the RNA at various locations such that portions of the RNA melt off. Accordingly, the "enzyme having RNase H activity" may be the RNase H activity of the reverse transcriptase or the RNase H activity of a separate enzyme such as, for example, *E*. *coli* RNase H, or both. In this respect, the method of the invention contemplates transcriptases having RNase H activity, such as AMV-RT or MMLV-RT, and other enzymes having DNA polymerase activity together with RNase H activity, such as, for example, *E*. *coli* DNA polymerase I (Holoenzyme), but also *E*. *coli* RNase H. The person skilled in the art will appreciate that with regard to RNAse H activity, the conditions, which may be determined experimentally, are chosen such that in most, and preferentially substantially all cases the oligonucleotide will remain hybridized to the target RNA, while the RNA sequence 3' from the target RNA, e.g. the poly-A-tail, will melt off.

The conditions under which the reactions should be performed are the normal conditions, i. e. buffer constitutions and temperatures, known by the person skilled in the art to be optimal for the mix of enzymes used. Alternatively, the person skilled in the art will be able by routine experimentation to determine applicable and/or optimal conditions. The terms "dNTPs" and "rNTPs" relate to nucleotides, i.e. deoxyribonucleotide triphosphate and ribonucleotide triphosphate, respectively. The present invention contemplates the use of modified forms of these nucleotides as well, known to the person skilled in the art, provided that these modified forms are recognised by the enzymes having DNA polymerase activity and/or the enzyme having RNA polymerase activity. Examples of modified forms, are for instance labelled nucleotides, such as labelled "rNTPs". Examples of non-naturally occurring bases that are capable of forming base-pairing relationships include, but are not limited to, aza- and deaza-pyrimidine analogues, aza- and deaza-purine analogues, and other heterocyclic base analogues, wherein one or more of the carbon and nitrogen atoms of the purine and pyrimidine rings have been substituted by heteroatoms, e.g., oxygen, sulphur, selenium, phosphorus, and the like.

If the interest exists to make an expression profile of just a few cells, the above described amplification may not yield enough copies of the RNA, for example to generate a signal if the copies are labelled. In certain special cases the RNA may need to be amplified further without introducing selectivity, thus again avoiding i.e. cDNA synthesis. There are multiple solutions to this problem, all of which are transcription based.

Accordingly, the present invention relates to a method, wherein the generated RNA is used as input material for further amplification. The newly synthesized RNA may now be further amplified by the following method. To the 3' end of every RNA molecule a double stranded promoter sequence is ligated by using RNA ligase. Since all 3'ends are chemically identical, there is no selectivity. The ligated promoter is used to initiate a second round of transcription generating more (labelled) RNA copies. As such, the RNAs generated by this second round of transcription are complementary to RNA copies generated in methods described *supra,* and thus are of the same strandness as the target RNA.

Accordingly, the present invention relates to a method, wherein the generated RNA is contacted with an RNA ligase, a double stranded nucleic acid complex comprising a double stranded DNA promoter sequence that can be recognized by an RNA polymerase, whereby one strand of said complex has a stretch of RNA attached to the 5' end of one of the DNA strands, an enzyme having RNA polymerase activity, and sufficient amounts of dNTPs and rNTPs; wherein the resulting reaction mixture is maintained under the appropriate conditions for a sufficient amount of time for the enzymatic processes to take place.

The procedure wherein the ligase is used may be performed as a separate reaction. That is, after RNA copies have been generated in a procedure like the one depicted in Figure 1, the RNA copies may be transferred to another reaction medium and subjected to the second reaction.

When all enzymes and the oligonucleotide and the promoter construct are combined with the initial reaction mixture a continuous process may even be obtained.

The components necessary for the further amplification may be comprised in the reaction mixture as described before, i.e. oligonucleotides, enzymes having DNA polymerase activity, enzymes having RNAse H activity, enzymes having RNA polymerase activity and sufficient amounts of dNTPS and rNTPs.

Accordingly, the present invention relates to a method wherein the reaction mixture as described before further comprises, an RNA ligase, and a double stranded nucleic acid complex comprising a double stranded DNA promoter sequence that can be recognized by the RNA polymerase, whereby one strand of said complex has a stretch of RNA attached to the 5' end of one of the DNA strands.

Again, one or more of the nucleotides used may be labelled.

Due to the orientation of the RNA polymerase promoter sequence, the RNA template is used in the second reaction to generate new sense strand RNA molecules. Typically, 10 to 1000, or 100 to 500 copies of each RNA is being made in the transcription reaction by the RNA polymerase.

The stretch of RNA attached to the 5' end of one of the DNA strands may be phosphorylated at the 5' end. Phosporylation enables the 5' end to be ligated. For instance, Bacteriophage T4 polynucleotide kinase may catalyze the transfer of y-phosphate of ATP to a 5' terminus of the RNA.

The promoter may be the same as in the first part of the procedure. For instance, the T7-promoter sequence may be used and the RNA polymerase then is T7 RNA polymerase.

In case the hybridising sequence is a predetermined sequence, and this predetermined sequence is a poiy-T stretch, the sense RNA made in this second round of transcription contains again a poly-A stretch at the 3' end making it possible to perform multiple cycles of amplification by repeatedly performing the method as illustrated by Figure 1 and the method using the ligase as described above.

Another method to further enhance the amplification factor of the non-biased mRNA amplification method is by adding a poly-A nucleotide stretch to the 3' end of the newly synthesized RNA. The poly-A nucleotide stretch may be added by the enzyme poly-A polymerase. To this added poly-A nucleotide stretch, an oligonucleotide, encompassing an oligo-T stretch as hybridising sequence and a promoter sequence, such as, for instance, a T7 promoter sequence, can hybridise. The previously described process may take place again. As a result, RNA will again be made by the transcription process and this newly synthesized RNA will be identical (for the large part) to the original target RNA that the whole reaction started with in the first place. One skilled in the art understands that the oligonucleotide, encompassing a poly-T stretch and a promoter sequence, e.g. a T7 promoter sequence, can also hybridise again to this RNA and now the process has entered in a continuous process of RNA synthesis by transcription, oligonucleotide annealing and double stranded promoter synthesis.

The procedure in which the poly-A-polymerase is added may be performed as a separate reaction. That is, after RNA copies have been made in a procedure like the one depicted in Figure 1, the RNA copies may be transferred to another reaction medium and subjected to the reaction which comprises the poly-A-polymerase, starting a continuous amplification process.

When the poly-A-polymerase, and the applicable oligonucleotide, is added to the initial reaction mixture the continuous amplification process may even start immediately from the original target RNA template.

Thus, the present invention relates to a method for generating multiple RNA copies as described before, wherein the generated RNA copies are contacted with poly-A polymerase, and possibly with a sufficient amount of rATP (ribo-adenosine triphosphate). The present invention also contemplates a method as described before, wherein the sample comprising target RNA is simultaneously contacted with poly-A-polymerase, at least one oligonucleotide encompassing an oligo-T-stretch and a promoter sequence, e.g. a T7 promoter sequence, an enzyme having DNA polymerase activity, an enzyme having RNase H activity, an enzyme having RNA polymerase activity and the necessary nucleotides. The resulting reaction mixture is maintained under appropriate conditions for a sufficient amount of time for the amplification to take place. ln the mix one or more nucleotides used may be labelled.

Due to the position of the newly added poly-A-stretch (3' end of the RNA molecule) the RNA polymerase will generate RNA of the opposite strandness, i.e. complementary to the template the RNA polymerase has used. The oligonucleotide, encompassing an oligo-T-stretch and promoter sequence, e.g. T7 promoter sequence, may be the same as in the first part of the procedure.

It will be appreciated that the nucleotides, e.g. dNTPs and rNTPs, used in the method of the invention may be labelled. Virtually any label that produces a detectable, quantifiable signal and that is capable of being attached to a nucleotide and incorporated into the generated RNA copy, can be used in conjunction with the methods of the invention. Suitable labels include, by way of example and not limitation, radioisotopes, fluorophores, chromophores, chemiluminescent moieties, etc. Preferably, the position of the label will not interfere with generation, hybridisation, detection or other post-hybridisation modifications of the labelled polynucleotide. A variety of different protocols may be used to generate the labelled nucleic acids, as is known in the art, where such methods typically rely on the enzymatic generation of labelled nucleic acid using a labelled nucleotide. For instance, label can be incorporated into the nucleic acid during the amplification steps in order to produce labelled target. Alternatively, the generated RNA copies may be labelled after these steps.

A variety of different labels may be employed, where such labels include fluorescent labels, isotopic labels, enzymatic labels, particulate labels, etc. For example, suitable isotopic labels include radioactive labels, e.g. ³²P, ³³P, ³⁵S, ³H. Other suitable labels include size particles that possess light scattering.

Genetic information is critical in the continuation of life processes. It has become very important to determine the genetic sequences of nucleotides which encode enzymes, structural proteins, and other effectors of biological functions. A gene expression pattern provides information about a particular cell. For instance, a comparison of the gene expression pattern of a normal cell with a suspected tumour cell, provides critical information on the stage and grade of the tumour. Methods for analysing tumour cells may employ gene expression analysis of samples. Gene expression patterns are formed and compared to reference patterns. Methods employing gene expression patterns provide improved accuracy as well as alternative basis for analysis from diagnostic an prognostic tools currently available. In particular, arrays, such as microarrays, find use in the analysis of differential gene expression, where the expression of genes in different cells, normally a cell of interest and a control (e.g. the predetermined pattern of expression), is compared and any discrepancies in expression are identified. In such assays, the presence of discrepancies indicates a difference in the classes of genes expressed in the cells being compared.

In methods of differential gene expression, arrays find use by serving as a substrate to which specific recognition reagents, such as, for example, polynucleotides, are bound. One then obtains target RNA or the RNA copies generated according to the method of the present invention from analogous cells, tissues or organs of, e.g. a healthy and diseased organism. The target RNA or RNA copies are next hybridised to the immobilized set of polynucleotide "probe" fragments. Differences between the resultant hybridisation patterns are subsequently detected and related to differences in gene expression in the two sources.

Accordingly, the present invention relates to a method for determining differences in gene expression in cell samples, comprising the steps of creating multiple RNA copies of one or more target RNA species according to the method of the invention, whereby a first pattern of expression is formed from the sample; comparing said first pattern of expression with a predetermined pattern of expression, e.g. an expression pattern in a control cell, whereby differences in gene expression are determined.

For improved reproducibility and accuracy of procedures, an automated system for determining gene expression profiles is contemplated. In particular, the present invention connotes the use of a probe array which is interrogated with the multiple RNA copies provided by the methods of the invention. The term "probe array" relates to a substrate having a high density matrix pattern of positionally defined specific recognition reagents. The multiple RNA copies provided by the method of the invention are capable of interacting, e.g. hybridising, with their specific counterparts, i.e. the specific recognition reagents, on the array. Because the specific recognition reagents are positionally defined, the sites of interaction will define the specificity of each interaction. The specific recognition reagents will typically be oligonucleotide probes, in which case said probe array is known as an oligonucleotide array.

The term "nucleic acid" as used herein means a polymer composed of nucleotides, e.g. deoxyribonucleotides or ribonucleotides. The terms "ribonucleic acid" and "RNA" as used herein means a polymer composed of ribonucleotides. The terms "deoxyribonucleic acid" and "DNA" as used herein means a polymer composed of deoxyribonucleotides. The term "oligonucleotide" as used herein denotes single stranded nucleotide multimers of from about 10 to about 100 nucleotides in length, or 5, 6, 7, 8, 9, 10, 11, 12, 20, 40, 60 or 80 nucleotides in length. The term "polynucleotide" as used herein refers to single or double stranded polymer composed of nucleotide monomers of from about 10 to about 100 nucleotides in length, usually of greater than about 100 nucleotides in length up to about 1000 nudeotides in length. Nevertheless, the polynucleotides may be relatively short, such as, for example, having a length of approximately 6, 7, 8, 9, 10, 11, 12, 20, 40, 60, 80, or 100 nucleotides.

The arrays of the present invention may be of any desired size, from two spots to 10⁶ spots or even more. The upper and lower limits on the size of the substrate are determined solely by the practical considerations of working with extremely small or large substrates.

For a given substrate size, the upper limit is determined only by the ability to create and detect the spots in the array. The preferred number of spots on a array generally depends on the particular use to which the array is to be put For example, mutation detection may require only a small array. In general, arrays contain from 2 to about 10⁶ spots, or from about 4 to about 10⁵ spots, or from about 8 to about 10⁴ spots, or between about 10 and about 2000 spots, or from about 20 to about 200 spots.

The immobilized polynucleotides on an array may be as few as four, or as many as hundreds, or even more, nucleotides in length. Contemplated as polynucleotides according to the invention are nucleic acids that are typically referred to in the art as oligonucleotides and also those referred to as nucleic acids. Thus, the arrays of the present invention are useful in applications where the generated RNA copies acids are hybridised to immobilized arrays of relatively short (such as, for example, having a length of approximately 6, 8, 10, 20, 40, 60, 80, or 100 nucleotides) probes.

In an embodiment, the set of polynucleotides on an array may correspond to particular mutations that are to be identified in a known sequence. For example, if a particular nucleic acid is known to contain an unidentified mutation at a particular position, then the mutated position can be identified with an array of eight polynucleotides, three corresponding to the three possible substitutions at that position, one corresponding to the deletion of the base at that position, and four corresponding to the insertion of the four possible bases at that position. Alternatively, for a known gene that may contain any of several possible identified mutations, the array can comprise polynucleotides corresponding to the different possible mutations. This embodiment is, for instance, useful for genes like oncogenes and tumour suppressors, which frequently have a variety of known mutations in different positions. Using arrays facilitates determining whether or not these genes contain mutations by allowing simultaneous screening with RNA copies of the present invention corresponding to each of these different positions.

The polynucleotides can be immobilized on the substrate using a wide variety of techniques. For example, the polynucleotides can be adsorbed or otherwise non-covalently associated with the substrate (for example, immobilization to nylon or nitrocellulose filters using standard techniques); they may be covalently attached to the substrate; or their association may be mediated by specific binding pairs, such as biotin and streptavidin.

A number of materials suitable for use as substrates in the instant invention have been described in the art. Exemplary suitable materials include, for example, acrylic, styrene-methyl methacrylate copolymers, ethylene/acrylic acid, acrylonitrile-butadienestyrene (ABS), ABS/polycarbonate, ABS/polysulfone, ABS/polyvinyl chloride, ethylene propylene, ethylene vinyl acetate (EVA), nitrocellulose, nylons (including nylon 6, nylon 6/6, nylon 6/6-6, nylon 6/9, nylon 6/10, nylon 6/12, nylon 11 and nylon 12), polycarylonitrile (PAN), polyacrylate, polycarbonate, polybutylene terephthalate (PBT), polyethylene terephthalate (PET), polyethylene (including low density, linear low density, high density, cross-linked and ultra-high molecular weight grades), polypropylene homopolymer, polypropylene copolymers, polystyrene (including general purpose and high impact grades), polytetrafluoroethylene (PTFE), fluorinated ethylene-propylene (FEP), ethylene-tetrafluoroethylene (ETFE), perfluoroalkoxyethylene (PFA), polyvinyl fluoride (PVF), polyvinylidene fluoride (PVDF), polychlorotrifluoroethylene (PCTFE), polyethylene-chlorotrifluoro-ethylene (ECTFE), polyvinyl alcohol (PVA), silicon styreneacrylonitrile (SAN), styrene maleic anhydride (SMA), and glass.

Other exemplary suitable materials for use as substrates in the arrays of the present invention include metal oxides. Metal oxides provide a substrate having both a high channel density and a high porosity, allowing high density arrays comprising different specific recognition reagents per unit of the surface for sample application. In addition, metal oxides are highly transparent for visible light. Metal oxides are relatively cheap substrates that do not require the use of any typical microfabrication technology and, that offers an improved control over the liquid distribution over the surface of the substrate, such as electrochemically manufactured metal oxide membrane. Metal oxide membranes having through-going, oriented channels can be manufactured through electrochemical etching of a metal sheet. Metal oxides considered are, among others, oxides of tantalum, titanium, and aluminium, as well as alloys of two or more metal oxides and doped metal oxides and alloys containing metal oxides. The metal oxide membranes are transparent, especially if wet, which allows for assays using various optical techniques. Such membranes have oriented through-going channels with well controlled diameter and useful chemical surface properties. Patent application EP-A-0 975 427 is exemplary in this respect, and is specifically incorporated in the present invention.

Accordingly, the present invention relates to a method as described herein, wherein the array is a flow-through micro array, the substrate is a porous substrate, such as an electrochemically manufactured metal oxide membrane, such as, for instance, aluminium oxide.

Variations to the above methods are appreciated by the person skilled in the art.

Moreover, the present invention relates to kits, which comprise compounds for use in the methods of the invention. Accordingly, the present invention relates to a kit for generating multiple RNA copies comprising an oligonucleotide comprising at its 5' side (possibly at its 5' end) a promoter sequence recognized by an RNA polymerase, wherein each oligonucleotide further comprises a target hybridising sequence, which is a random sequence or a predetermined sequence, a modification at its 3' terminal end in such a way that extension there from is prohibited and at least one chimeric linkage between nucleotides at the 3' end, wherein said chimeric linkage may contain at least one phosphorothioate linkage between nucleotides, or PNA, LNA or GRipNA backbone; and, possibly, an enzyme having DNA polymerase activity, possibly, an enzyme having RNase H activity, possibly, an enzyme having RNA polymerase activity, and, possibly, sufficient amounts of dNTPs and rNTPs; and, instructions to carry out the method for generating multiple RNA copies.

In addition, the present invention relates to a kit as described above, which further comprises an RNA ligase, and a double stranded nucleic acid complex comprising a double stranded DNA promoter sequence that can be recognized by an RNA polymerase, whereby one strand of said complex has a stretch of RNA attached to the 5' end of one of the DNA strands, and instructions to carry out further amplification.

The present invention relates also to the kits as described above, which further comprises a probe array, and possibly instructions to interrogate the array.

Before the subject invention is described further, it is to be understood that the invention is not limited to the particular embodiments of the invention described herein, as variations of the particular embodiments may be made and still fall within the scope of the appended claims. It is also to be understood that the terminology employed is for the purpose of describing particular embodiments, and is not intended to be limiting. Instead, the scope of the present invention will be established by the appended claims.

The following examples are offered by way of illustration and not by way of limitation.

### EXAMPLES

### Example 1: random primed-tyras reaction 1

100 ng of in vitro transcribed RNA (pGEMExpress Positive Control Template transcribed according to protocol TM0126 of Promega) is used as a template in the random primed-tyras reaction 1. This reaction contains 3.6 µl water with 100 ng of this template, 4 µl 5XNN buffer (Tris-HCI 200mM, pH 8.5; MgCl₂ 60mM; KCl 350 mM; DTT 25 mM; dNTP's 5mM of each; rATP, rCTP and GTP, 2mM of each), 1.8 µl 100mM rUTP and 1.6 µl of 10mM Flu-UTP, 4 µl primer mix [37.5 µl 100% DMSO, 5 µl of a 100 µM oligonucleotide with the sequence:
(of which the last six nucleotides represent a random hexamer -N stands for an equimolar mixture of A,C,G and T bases- of which the last nucleotide is a 2'-3' dideoxynucleotide and the final phosphate bond has been exchanged with a phosphorothioate bond) and 7.5 µl water for a total volume of 50 µl].

This reaction is incubated at 65 °C for 5 minutes and subsequently at 41 °C for 5 minutes. Then 5 µl enzyme mix (sorbitol 1.5M; BSA 2.1 µg; T7 RNA polymerase 32 units and AMV-reverse transcriptase 25.3 units) is added to the reaction and gently mixed by tapping the tube. After a short incubation of 5 minutes at 41 °C, the tubes are briefly spun in a centrifuge to collect droplets and then the reaction is incubated for another 120 minutes at 41 °C.

Then, the labeled reaction products are isolated using the RNEasy kit according to the manufacturer's protocol (Qiagen) and eluted in 30 µl of water.

1 µg of the template (pGEMExpress Positive Control template, Promega) is denatured for 5 minutes by holding the tube in which it is contained in a boiling water bath. After cooling the template is spotted onto a membrane (Hybond Nplus, Amersham Pharmacia Biotech) next to a spot containing similarly treated Luciferase SP6 Control DNA (Promega) as an appropriate control. After drying the spots in the air and treating the membrane with UV light as described in the manufacturer's protocol the membrane is hybridized with the labelled reaction product following the protocol as delivered with the Dig Wash and Block buffer set (Roche). The probe used in this hybridization is the labelled reaction product that has been treated for 5 minutes at 95 °C immediately before use. After the hybridization, the membrane is incubated with 15 units of Anti-Fluorescein Alkaline Phosphatase conjugated Fab fragments (Roche) and subsequently developed with one tablet of NBT/BCIP as substrate, according to the manufacturer's protocol.

By comparing the strength of the signals by eye it is obvious that more substrate is deposited on the spotted pGEMExpress template than on the control spot reflecting the hybridization of the labelled reaction product to its template.

### Example 2: random primed-tyras reaction 2

100 ng in vitro transcribed RNA (Luciferase SP6 Control DNA, restricted with Sacl and transcribed according to protocol TM0126 of Promega) was used as a template in the random primed-tyras reaction 2. This reaction contained (end concentration is indicated) Tris-HCI 40mM, pH 8.5, MgCl₂ 16 mM, KCl 30 mM, DTT 50 mM, sorbitol 375 mM, BSA 2.0 mg, dNTP's 5mM each, rATP, rCTP, rGTP, 2mM each, rUTP 1.8 mM, Flu-UTP 0.2 mM and an oligonucleotide with sequence:
of which the last six nucleotides represent a random hexamer, -N stands for an equimolar mixture of A, C, G and T bases- of which the last nucleotide is a 2'-3' dideoxynucleotide) 10 µM in an end volume of 9 µl.

This reaction was incubated for 15 minutes at 30 °C, after which the temperature was increased to 37 °C. Immediately thereafter, 6 units AMV-reverse transcriptase were added to the reaction and the mixture was gently mixed by tapping the tube. After an incubation for 15 minutes at 37 °C the tubes were briefly spun in a centrifuge to collect droplets and then 40 *µ*l of a solution containing 3.2 *µ*l of a 25 mM rNTP mix, 2 *µ*l of 100 mM DTT, 1.6 *µ*l of 1 mM Tris/acetaat pH 8.5, 5 *µ*l of 100mM Mg₂Cl, 30 units T7 RNA polymerase and 16.55 *µ*l water were added. This reaction was incubated for 120 minutes at 37 °C.

The labeled reaction product was isolated using the RNEasy kit according to the manufacturers protocol (Qiagen) and eluted in 30 *µ*l of water. Five microliter of this eluate was analysed on a 1% agarose gel in a 0.5 x TAE buffer (I-mupid system, Cosmo Bio Co Ltd) with 5 *µ*l of loading buffer 11 (Ambion, cat nr 8546). The sample was heated for 5 minutes at 70°C before loading the gel. The gel was run until the lower blue band crossed three quarter of the gel. Incorporation of Flu-UTP was validated by visualization on a common UV transilluminator at 302 nm. A smear was visible with an length until approximately the length of the input template.

The gel was blotted onto an Hybond N+membrane (Amersham) with the Turboblotter (Schleicher & Schuell) using the recommended procedure by the manufacturer. The blot was hybridized using a standard oligo hybridization procedure, and ingredients (Roche; art. nrs 1603558 and 1585762) with a 3'-DNP derived oligonucleotide as a probe in an end concentration of 2.5 nM, of which the sequence was identical to a stretch of the luciferase sequence in the plasmid described above. The 30 nucleotides long probe has the sequence:

Hybridization took place at 37 °C overnight under constant shaking (50rpm). The blot was developed after a 0.2 x SSC wash (20 x SSC = 175.3 g NaCl, 88.2 g Sodium citrate per liter, pH 7.0) for 10 minutes at 50 °C using the recommended procedure by the manufacturer, using anti-DNP/AP (Dako cat# D5103) and as detection solution a NBT/BCIP tablet (Roche cat # 1697471).

The blot showed a smear after hybridization with a length corresponding to the smear seen after UV illumination, proving the specificity of this random primed-tyras reaction.

## Claims

1. A method for generating multiple RNA copies comprising the steps of:
(a) providing a sample comprising target RNA, wherein said sample is simultaneously contacted with:
- an oligonucleotide comprising at its 5' side a promoter sequence recognized by an RNA polymerase, wherein said oligonudeotide further comprises:
- a target hybridising sequence, which is a random sequence,
- a modified nudeotide at its 3' terminal end in such a way that extension therefrom is prohibited, wherein said modified nucleotide is chosen from the group comprising nucleotides comprising alkane-diol residues, cordycepins, amino-alkyls, and dideoxynucleotides,
- at least one chimeric linkage between nucleotides at the 3' end; wherein said chimeric linkage may contain at least one phosphorothioate linkage between nucleotides, or a PNA, LNA or GripNA backbone, and;
- an enzyme having DNA polymerase activity;
- an enzyme having RNase H activity;
- an enzyme having RNA polymerase activity; and,
- sufficient amounts of nucleotides; and,
(b) maintaining the resulting reaction mixture under the appropriate conditions for a sufficient amount of time for the enzymatic processes to take place.

2. A method for generating multiple RNA copies comprising the steps of:
(a) providing a sample comprising target RNA, wherein said sample is simultaneously contacted with:
- a DNA oligonucleotide comprising at its 5' side a promoter sequence recognized by an RNA polymerase, wherein said oligonucleotide further comprises:
- a target hybridising sequence, which is a predetermined sequence,
- a modified nudeotide at its 3' terminal end in such a way that extension therefrom is prohibited, wherein said modified nudeotide is chosen from the group comprising nudeotides comprising alkane-diol residues, cordycepins, amino-alkyls, and dideoxynudeotides,
- at least one chimeric linkage between nudeotides at the 3' end; wherein said chimeric linkage may contain at least one phosphorothioate linkage between nudeotides, or a PNA, LNA or GripNA backbone, and;
- an enzyme having Klenow pol I exo (-) activity;
- an enzyme having RNase H activity;
- an enzyme having RNA polymerase activity; and,
- sufficient amounts of nucleotides; and,
(b) maintaining the resulting reaction mixture under the appropriate conditions for a suffident amount of time for the enzymatic processes to take place.

3. A method for generating multiple RNA copies comprising the steps of.
(a) providing a sample comprising target RNA; wherein said sample is simultaneously contacted with:
- an oligonucleotide comprising at its 5' side a promoter sequence recognized by an RNA polymerase, wherein said oligonucleotide further comprises:
- a target hybridising sequence, wherein said hybridising sequence is a predetermined sequence,
- a modified nudeotide at its 3' terminal end in such a way that extension therefrom is prohibited, wherein said modified nudeotide is chosen from the group comprising nucleotides comprising alkane-diol residues, cordycepins, amino-alkyls, and dideoxynucleotides,
- at least one chimeric linkage between nucleotides at the 3' end; wherein said chimeric linkage may, contain at least one phosphorothioate linkage between nudeotides, or a PNA, LNA or GripNA backbone, and,
- an enzyme having DNA polymerase activity;
- an enzyme having RNase H activity;
- an enzyme having RNA polymerase activity; and,
- sufficient amounts of nucleotides; and,
(b) maintaining the resulting reaction mixture under the appropriate conditions for a sufficient amount of time for the enzymatic processes to take place.

4. The method according to any of claims 1 to 3, wherein said target RNA is of eukaryotic; prokaryotic or viral origin, or a mixture thereof.

5. The method according to any of claims 1 to 4, wherein said target RNA is chosen from the group comprising total RNA, mRNA, cRNA, rRNA, tmRNA, asRNA, hnRNA or tRNA, including any combination thereof.

6. The method according to any of claims 2 to 5, wherein said predetermined sequence is chosen from the group comprising gene-specific sequences, viral sequences, prokaryotic sequences, mutation-specific sequences, poly-T sequences, genomic sequences and rRNA.

7. The method according to any of claims 1 to 6, wherein at least one of the nucleotides, e.g. dNTPs and rNTPs, is provided with a label.

8. The method according to any of claims 1 to 7, wherein the generated RNA is used as input material for further amplification.

9. The method according to any of claims 1 to 8, wherein the generated RNA is contacted with:
- an RNA ligase,
- a double stranded nudeic acid complex comprising a double stranded DNA promoter sequence that can be recognized by an RNA polymerase, whereby one strand of said complex has a stretch of RNA attached to the 5' end of one of the DNA strands,
- an enzyme having RNA polymerase activity, and
- sufficient amounts of dNTPs and rNTPs;
wherein the resulting reaction mixture is maintained under the appropriate conditions for a sufficient amount of time for the enzymatic processes to take place.

10. The method according to any of claims 1 to 9, wherein the reaction mixture further comprises:
- an RNA ligase; and,
- a double stranded nucleic acid complex comprising a double stranded DNA promoter sequence that can be recognized by the RNA polymerase, whereby one strand of said complex has a stretch of RNA attached to the 5' end of one of the DNA strands.

11. The method according to any of claims 1 to 10, wherein the generated RNA copies are contacted with poly A polymerase.

12. The method according to any of the claims 1 to 11, wherein the starting material is simultaneously contacted with a poly A polymerase.

13. The method according to any of claims 1 to 12, wherein said promoter sequence is a T7 promoter sequence.

14. The method according to any of claims 1 to 13, wherein said RNA polymerase is a T7 RNA polymerase.

15. The method according to any of claims 1 and 3 to 14, wherein said enzyme having DNA polymerase activity is AMV-RT or MMLV RT.

16. The method according to any of claims 1 to 15, wherein said enzyme having RNase H activity is *E. coli* RNase H.

17. The method according to any of claims 1 to 16, wherein said enzyme having RNase H activity is reverse transcriptase.

18. The method according to claim 17, wherein said enzyme having RNase H activity is AMV-RT or MMLV-RT.

19. A method for determining differences in gene expression in cell samples, comprising the steps of:
- creating multiple RNA copies of one or more target RNA species according to the method of any of claims 1 to 18, whereby a first pattern of expression is formed from the sample;
- comparing said first pattern of expression with a predetermined pattern of expression, whereby differences in gene expression are determined.

20. The method according to any of claims 1 to 19, wherein said multiple RNA copies are used to interrogate a probe array.

21. The method according to claim 20, wherein said probe array is an oligonucleotide array.

22. Kit for generating multiple RNA copies comprising:
an oligonucleotide comprising at its 5' side a promoter sequence recognized by an RNA polymerase, wherein said oligonucleotide further comprises a target hybridising sequence, which is a random sequence or a predetermined sequence, said predetermined sequence complexing predominantly with the intended target RNA to be amplified, a modification at its 3' terminal end in such a way that extension therefrom is prohibited, and at least one chimeric linkage between nucleotides at the 3' end, wherein said chimeric linkage may contain at least one phosphorothioate linkage between nucleotides, or PNA, LNA or GripNA backbone, and
- instructions to carry out the method according to any of the Claims 1 to 21 for generating multiple RNA copies.

23. The kit according to claim 22, further comprising:
- an RNA ligase,
- a double stranded nucleic add complex comprising a double stranded DNA promoter sequence that can be recognized by an RNA polymerase, whereby one strand of said complex has a stretch of RNA attached to the 5' end of one of the DNA strands, and
- instructions to carry out further amplification.

24. The kit according to claim 22 or 23, further comprising a probe array.

## Patentansprüche

1. Verfahren zur Erzeugung multipler RNA-Kopien, wobei das Verfahren die folgenden Schritte umfasst:
(a) Bereitstellen einer Ziel-RNA-umfassenden Probe, wobei die Probe gleichzeitig in Kontakt gebracht wird mit:
- einem Oligonukleotid, das an seiner 5'-Seite eine Promotorsequenz umfasst, die durch eine RNA-Polymerase erkannt wird, wobei das Oligonukleotid weiter Folgendes umfasst:
- eine hybridisierende Zielsequenz, die eine zufällige Sequenz ist,
- ein an seinem 3'-terminalen Ende solchermaßen modifiziertes Nukleotid, dass eine von ihm ausgehende Extension verhindert wird, wobei das modifizierte Nukleotid aus Nukleotiden ausgewählt wird, die Alkandiol-Reste, Cordyzepine, Aminoalkyle und Didesoxynukleotide umfassen,
- wenigstens eine chimere Verknüpfung zwischen Nukleotiden am 3'-Ende;
wobei die chimere Verknüpfung wenigstens eine Phosphorthioatverknüpfung zwischen Nukleotiden oder ein PNA-, LNA- oder GripNA-Rückgrat enthalten kann, und;
- einem Enzym mit DNA-Polymeraseaktivität;
- einem Enzym mit RNase H-Aktivität;
- einem Enzym mit RNA-Polymerase-Aktivität; und,
- ausreichenden Mengen von Nukleotiden; und
(b) Halten des resultierenden Reaktionsgemisches unter geeigneten Bedingungen für eine ausreichende Menge an Zeit, so dass die enzymatischen Vorgänge stattfinden.

2. Verfahren zur Erzeugung multipler RNA-Kopien, wobei das Verfahren die folgenden Schritte umfasst:
(a) Bereitstellen einer Ziel-RNA-umfassenden Probe, wobei die Probe gleichzeitig in Kontakt gebracht wird mit:
- einem DNA-Oligonukleotid, das an seiner 5'-Seite eine Promotorsequenz umfasst, die durch eine RNA-Polymerase erkannt wird, wobei das Oligonukleotid weiter Folgendes umfasst:
- eine hybridisierende Zielsequenz, die eine vorbestimmte Sequenz ist,
- ein an seinem 3'-terminalen Ende solchermaßen modifiziertes Nukleotid, dass eine von ihm ausgehende Extension verhindert wird, wobei das modifizierte Nukleotid aus Nukleotiden ausgewählt wird, die Alkandiol-Reste, Cordyzepine, Aminoalkyle und Didesoxynukleotide umfassen,
- wenigstens eine chimere Verknüpfung zwischen Nukleotiden am 3'-Ende;
wobei die chimere Verknüpfung wenigstens eine Phosphorthioatverknüpfung zwischen Nukleotiden oder ein PNA-, LNA- oder GripNA-Rückgrat enthalten kann, und;
- einem Enzym mit Klenow Pol I exo (-) -Polymeraseaktivität;
- einem Enzym mit RNase H-Älctivität;
- einem Enzym mit RNA-Polymerase-Aktivität; und,
- ausreichenden Mengen von Nukleotiden; und
(b) Halten des resultierenden Reaktionsgemisches unter geeigneten Bedingungen für eine ausreichende Menge an Zeit, so dass die enzymatischen Vorgänge stattfinden.

3. Verfahren zur Erzeugung multipler RNA-Kopien, wobei das Verfahren die folgenden Schritte umfasst:
(a) Bereitstellen einer Ziel-RNA-umfassenden Probe, wobei die Probe gleichzeitig in Kontakt gebracht wird mit:
- einem Oligonukleotid, das an seiner 5'-Seite eine Promotorsequenz umfasst, die durch eine RNA-Polymerase erkannt wird, wobei das Oligonukleotid weiter Folgendes umfasst:
- eine hybridisierende Zielsequenz, wobei die hybridisierende Sequenz eine vorbestimmte Sequenz ist,
- ein an seinem 3'-terminalen Ende solchermaßen modifiziertes Nukleotid, dass eine von ihm ausgehende Extension verhindert wird, wobei das modifizierte Nukleotid aus Nukleotiden ausgewählt wird, die Alkandiol-Reste, Cordyzepine, Aminoalkyle und Didesoxynukleotide umfassen,
- wenigstens eine chimere Verknüpfung zwischen Nukleotiden am 3'-Ende; wobei die chimere Verknüpfung wenigstens eine Phosphorthioatverknüpfung zwischen Nukleotiden oder ein PNA-, LNA- oder GripNA-Rückgrat enthalten kann, und;
- einem Enzym mit DNA-Polymeraseaktivität;
- einem Enzym mit RNase H-Aktivität;
- einem Enzym mit RNA-Polymerase-Aktivität; und,
- ausreichenden Mengen von Nukleotiden; und
(b) Halten des resultierenden Reaktionsgemisches unter geeigneten Bedingungen für eine ausreichende Menge an Zeit, so dass die enzymatischen Vorgänge stattfinden.

4. Verfahren gemäß einem der Ansprüche 1 bis 3, wobei die Ziel-RNA eukaryontischen, prokaryontischen oder viralen Ursprungs, oder eines Gemisches davon, ist.

5. Verfahren gemäß einem der Ansprüche 1 bis 4, wobei die Ziel-RNA aus der Gruppe ausgewählt wird, die Gesamt-RNA, mRNA, cRNA, rRNA, tmRNA, asRNA, hnRNA oder tRNA umfasst, einschließlich jeder Kombination davon.

6. Verfahren gemäß einem der Ansprüche 2 bis 5, wobei die vorbestimmte Sequenz aus der Gruppe ausgewählt wird, die genspezifische Sequenzen, virale Sequenzen, prokaryontische Sequenzen, mutationsspezifische Sequenzen, Poly-T-Sequenzen, genomische Sequenzen und rRNA umfasst.

7. Verfahren gemäß einem der Ansprüche 1 bis 6, wobei wenigstens eines der Nukleotide, z.B. dNTPs und rNTPs, mit einer Markierung versehen ist.

8. Verfahren gemäß einem der Ansprüche 1 bis 7, wobei die erzeugte RNA als Ausgangsmaterial für weitere Amplifikation verwendet wird.

9. Verfahren gemäß einem der Ansprüche 1 bis 8, wobei die erzeugte RNA in Kontakt gebracht wird mit:
- einer RNA-Ligase,
- einem doppelsträngigen Nukleinsäurekomplex, der eine doppelsträngige DNA-Promotorsequenz umfasst, die von einer RNA-Polymerase erkannt werden kann, wodurch ein Strang des Komplexes einen RNA-Anteil hat, der am 5'-Ende eines der DNA-Stränge angebracht ist,
- einem Enzym mit RNA-Polymerase-Aktivität, und
- ausreichenden Mengen von dNTPs und rNTPs;
wobei das resultierende Reaktionsgemisch eine ausreichende Zeit lang unter geeigneten Bedingungen gehalten wird, so dass die enzymatischen Vorgänge stattfinden.

10. Verfahren gemäß einem der Ansprüche 1 bis 9, wobei das Reaktionsgemisch weiter Folgendes umfasst:
- eine RNA-Ligase; und
- einen doppelsträngigen Nukleinsäurekomplex, der eine doppelsträngige DNA-Promotorsecluenz umfasst, die von der RNA-Polyznerase erkannt werden kann, wodurch ein Strang des Komplexes einen RNA-Anteil hat, der am 5'-Ende eines der DNA-Stränge angebracht ist.

11. Verfahren gemäß einem der Ansprüche 1 bis 10, wobei die erzeugten RNA-Kopien mit einer Poly-A-Polymerase in Kontakt gebracht werden.

12. Verfahren gemäß einem der Ansprüche 1 bis 11, wobei das Ausgangsmaterial gleichzeitig mit einer Poly-A-Polymerase in Kontakt gebracht wird.

13. Verfahren gemäß einem der Ansprüche 1 bis 12, wobei die Promotorsequenz eine T7-Promotorsequenz ist.

14. Verfahren gemäß einem der Ansprüche 1 bis 13, wobei die RNA-Polymerase eine T7-RNA-Polymerase ist.

15. Verfahren gemäß einem der Ansprüche 1 und 3 bis 14, wobei das Enzym mit DNA-Polymerase-Aktivität AMV-RT oder MMLV-RT ist.

16. Verfahren gemäß einem der Ansprüche 1 bis 15, wobei das Enzym mit RNase H-Aktivität *E. coli*-RNase H ist.

17. Verfahren gemäß einem der Ansprüche 1 bis 16, wobei das Enzym mit RNase H-Aktivität reverse Transkriptase ist.

18. Verfahren gemäß Anspruch 17, wobei das Enzym mit RNase H-Aktivität AMV-RT oder MMLV-RT ist.

19. Verfahren zur Bestimmung von Unterschieden in der Genexpression in Zellproben, wobei das Verfahren die folgenden Schritte umfasst:
- Erzeugung multipler RNA-Kopien von wenigstens einer Ziel-RNA-Spezies gemäß dem Verfahren eines der Ansprüche 1 bis 18, wodurch von der Probe ein erstes Expressionsmuster ausgebildet wird;
- Vergleichen des ersten Expressionsmusters mit einem vorbestimmten Expressionsmuster, wodurch die Unterschiede in der Genexpression bestimmt werden.

20. Verfahren gemäß einem der Ansprüche 1 bis 19, wobei die multiplen RNA-Kopien verwendet werden, um einen Sonden-Array abzufragen.

21. Verfahren gemäß Anspruch 20, wobei der Sonden-Array ein Oligonukleotid-Array ist.

22. Kit zur Erzeugung multipler RNA-Kopien, wobei das Kit Folgendes umfasst:
- ein Oligonukleotid, das an seiner 5'-Seite eine Promotorsequenz umfasst, die durch eine RNA-Polymerase erkannt wird, wobei das Oligonukleotid weiterhin eine hybridisierende Zielsequenz umfasst, die eine zufällige Sequenz oder eine vorbestimmte Sequenz ist, wobei die vorbestimmte Sequenz überwiegend mit der zur Amplifikation vorgesehenen Ziel-RNA einen Komplex ausbildet, und wobei das Oligonukleotid weiter eine Modifikation an seinem 3'-terminalen Ende solchermaßen umfasst, dass eine von ihm ausgehende Extension verhindert wird, und wenigstens eine chimere Verknüpfung zwischen Nukleotiden am 3'-Ende; wobei die chimere Verknüpfung wenigstens eine Phosphorthioatverknüpfung zwischen Nukleotiden oder ein PNA-, LNA- oder GripNA-Rückgrat enthalten kann, und
- Anweisungen, um das Verfahren gemäß einem der Ansprüche 1 bis 21 zur Erzeugung multipler RNA-Kopien, auszuführen.

23. Kit gemäß Anspruch 22, wobei das Kit weiter Folgendes umfasst:
- eine RNA-Ligase,
- einen doppelsträngigen Nukleinsäurekomplex, der eine doppelsträngige DNA-Promotorsequenz umfasst, die von einer RNA-Polymerase erkannt werden kann, wodurch ein Strang des Komplexes einen RNA-Anteil hat, der am 5'-Ende eines der DNA-Stränge angebracht ist, und
- Anweisungen, um weitere Amplifikation auszuführen.

24. Kit gemäß Anspruch 22 oder 23, weiter umfassend einen Sonden-Array.

## Revendications

1. Procédé pour générer des copies multiples d'ARN comprenant les étapes de :
(a) mettre à disposition un échantillon comprenant l'ARN cible, dans lequel ledit échantillon est simultanément mis en contact avec .
- un oligonucléotide comprenant sur son côté 5' une séquence promoteur reconnue par une ARN polymérase, dans lequel ledit oligonucléotide comprend de plus :
- une séquence d'hybridation cible qui est une séquence aléatoire,
- un nucléotide modifié à son extrémité terminale 3', de telle sorte que l'extension à partir de celle-ci est prohibée, dans lequel ledit nucléotide modifié est choisi dans le groupe comprenant des nucléotides comprenant des résidus alcane-diols, des cordycépines, des amino-alkyles et des didésoxynucléotides,
- au moins une liaison chimère entre des nucléotides à l'extrémité 3' ; où ladite liaison chimère peut contenir au moins une liaison phosphorothioate entre des nucléotides ou une ossature PNA, LNA ou GripNA, et
- une enzyme ayant une activité ADN polymérase;
- une enzyme ayant une activité RNase H ;
- une enzyme ayant une activité ARN polymérase; et
- des quantités suffisantes de nucléotides ; et
(b) maintenir le mélange réactionnel obtenu dans les conditions appropriées pendant une durée suffisante pour que les processus enzymatiques aient lieu.

2. Procédé pour générer des copies multiples d'ARN comprenant les étapes de :
(a) mettre à disposition un échantillon contenant l'ARN cible, ledit échantillon étant simultanément mis en contact avec :
- un oligonucléotide ADN comprenant sur son côté 5' une séquence promoteur reconnue par une ARN polymérase, ledit oligonucléotide comprenant de plus :
- une séquence d'hybridation cible qui est une séquence prédéterminée,
- un nucléotide modifié à son extrémité terminale 3', de telle sorte que l'extension à partir de celle-ci est prohibée, ledit nucléotide modifié étant choisi dans le groupe comprenant les nucléotides comprenant des résidus alcane-diols, des cordycépines, des amino-alkyles et des didésoxynucléotides,
- au moins une liaison chimère entre des nucléotides à l'extrémité 3' ; dans lequel ladite liaison chimère peut contenir au moins une liaison phosphorothioate entre des nucléotides ou une ossature PNA, LNA ou GripNA, et
- une enzyme ayant une activité Klenow pol I exo (-) ;
- une enzyme ayant une activité RNase H ;
- une enzyme ayant une activité ARN polymérase; et
- des quantités suffisantes de nucléotides ; et
(b) maintenir le mélange réactionnel obtenu dans les conditions appropriées pendant une durée suffisante pour que les processus enzymatiques s'effectuent.

3. Procédé pour générer des copies multiples d'ARN comprenant les étapes consistant à :
(a) mettre à disposition un échantillon comprenant l'ARN cible, ledit échantillon étant simultanément mis en contact avec :
- un oligonucléotide comprenant sur son côté 5' une séquence promoteur reconnue par une ARN polymérase, ledit oligonucléotide comprenant de plus :
- une séquence d'hybridation cible, ladite séquence d'hybridation étant une séquence prédéterminée,
- un nucléotide modifié à son extrémité terminale 3', de telle sorte que l'extension à partir de celle-ci est prohibée, ledit nucléotide modifié étant choisi dans le groupe comprenant des nucléotides comprenant des résidus alcane-diols, des cordycépines, des amino-alkyles et des didésoxynucléotides,
- au moins une liaison chimère entre des nucléotides à l'extrémité 3' ; ladite liaison chimère pouvant contenir au moins une liaison phosphorothioate entre des nucléotides ou une ossature PNA, LNA ou GripNA, et
- une enzyme ayant une activité ADN polymérase;
- une enzyme ayant une activité RNase H ;
- une enzyme ayant une activité ARN polymérase; et
- des quantités suffisantes de nucléotides ; et
(b) maintenir le mélange réactionnel obtenu dans les conditions appropriées pendant une durée suffisante pour que s'effectuent les processus enzymatiques.

4. Procédé selon l'une quelconque des revendications 1 à 3, dans lequel ledit ARN cible est d'origine eucaryote, procaryote ou virale ou leur mélange.

5. Procédé selon l'une quelconque des revendications 1 à 4, dans lequel ledit ARN cible est choisi dans le groupe comprenant ARN, ARNm, ARNc, ARNr, ARNtm, ARNas, ARNhn ou ARNt, incluant toute combinaison de ceux-ci.

6. Procédé selon l'une quelconque des revendications 2 à 5, dans lequel ladite séquence prédéterminée est choisie dans le groupe comprenant des séquences spécifiques aux gènes, des séquences virales, des séquences procaryotes, des séquences spécifiques de mutations, des séquences poly-T, des séquences génomiques et de l'ARNr.

7. Procédé selon l'une quelconque des revendications 1 à 6, dans lequel au moins l'un des nucléotides, par exemple dNTPs et rNTPs, est doté d'un marqueur.

8. Procédé selon l'une quelconque des revendications 1 à 7, dans lequel l'ARN généré est utilisé comme matériau d'entrée pour l'amplification subséquente.

9. Procédé selon l'une quelconque des revendications 1 à 8, dans lequel l'ARN généré est mis en contact avec :
- une ARN ligase,
- un complexe d'acide nucléique à double brin comprenant une séquence promoteur ADN double brin qui peut être reconnue par une ARN polymérase, de manière à ce qu'un brin dudit complexe reçoive une extension d'ARN fixée sur l'extrémité 5' de l'un des brins d'ADN,
- une enzyme ayant une activité ARN polymérase, et
- des quantités suffisantes de dNTPs et rNTPs ;
dans lequel le mélange réactionnel obtenu est maintenu dans les conditions appropriées pendant une durée suffisante pour que s'effectuent les processus enzymatiques.

10. Procédé selon l'une quelconque des revendications 1 à 9, dans lequel le mélange réactionnel comprend de plus :
- une ARN ligase; et
- un complexe d'acide nucléique à double brin comprenant une séquence promoteur ADN double brin qui peut être reconnue par l'ARN polymérase, de manière à ce qu'un brin dudit complexe reçoive une extension d'ARN fixée sur l'extrémité 5' de l'un des brins d'ADN.

11. Procédé selon l'une quelconque des revendications 1 à 10, dans lequel les copies ARN générées sont mises en contact avec la poly-A polymérase.

12. Procédé selon l'une quelconque des revendications 1 à 11, dans lequel le matériau de départ est simultanément mis en contact avec une poly-A polymérase.

13. Procédé selon l'une quelconque des revendications 1 à 12, dans lequel ladite séquence promoteur est une séquence promoteur T7.

14. Procédé selon l'une quelconque des revendications 1 à 13, dans lequel ladite ARN polymérase est une ARN polymérase T7.

15. Procédé selon l'une quelconque des revendications 1 et 3 à 14, dans lequel ladite enzyme ayant une activité d'ADN polymérase est la AMV-RT ou la MMLV-RT.

16. Procédé selon l'une quelconque des revendications 1 à 15, dans lequel ladite enzyme ayant une activité RNase H est la RNase H de E. coli.

17. Procédé selon l'une quelconque des revendications 1 à 16, dans lequel ladite enzyme ayant une activité RNase H est la transcriptase inverse.

18. Procédé selon la revendication 17, dans lequel ladite enzyme ayant une activité RNase H est la AMV-RT ou la MMLV-RT.

19. Procédé pour déterminer les différences dans l'expression de gènes dans des échantillons de cellules, comprenant les étapes de :
- créer des copies multiples d'ARN d'une ou de plusieurs espèces ARN cibles selon le procédé de l'une quelconque des revendications 1 à 18, dans lequel un premier motif d'expression est formé à partir de l'échantillon ;
- comparer ledit premier motif d'expression avec un motif prédéterminé d'expression de manière à déterminer les différences d'expression de gènes.

20. Procédé selon l'une quelconque des revendications 1 à 19, dans lequel lesdites copies multiples d'ARN sont utilisées pour interroger une série de sondes.

21. Procédé selon la revendication 20, dans lequel ladite série de sondes est une série d'oligonucléotide.

22. Kit pour générer des copies multiples d'ARN comprenant :
- un oligonucléotide comprenant sur son côté 5' une séquence promoteur reconnue par une ARN polymérase, ledit oligonucléotide comprenant de plus une séquence d'hybridation cible qui est une séquence aléatoire ou une séquence prédéterminée, ladite séquence prédéterminée se complexant de façon prédominante avec l'ARN cible devant être amplifié, une modification à son extrémité terminale 3' de telle sorte que l'extension à partir de celle-ci est prohibée, et au moins une liaison chimère entre les nucléotides sur l'extrémité 3', ladite liaison chimère pouvant contenir au moins une liaison phosphorothioate entre les nucléotides ou une ossature PNA, LNA ou GripNA, et
- des instructions pour effectuer le procédé selon l'une quelconque des revendications 1 à 21 pour générer des copies multiples d'ARN.

23. Kit selon la revendication 22, comprenant de plus :
- une ARN ligase,
- un complexe d'acide nucléique à double brin comprenant une séquence promoteur ADN à double brin qui peut être reconnue par une ARN polymérase, de manière à ce qu'un brin dudit complexe reçoive une extension d'ARN fixée sur l'extrémité 5' de l'un des brins d'ADN, et
- des instructions pour effectuer l'amplification subséquente.

24. Kit selon la revendication 22 ou 23, comprenant de plus une série de sondes.


### SEQUENCE LISTING

<110> PamGene B.V.
<120> Improved methods for generating multiple RNA copies
<130> PAM-011-PCT
<150> EP 02447204.5
   <151> 2002-10-30
<150> US 60/440,688
   <151> 2003-01-17
<160> 3
<170> PatentIn version 3.1
<210> 1
   <211> 55
   <212> DNA
   <213> Artificial Sequence
<220>
   <223> Primer
<220>
   <221> misc_feature
   <222> (50)..(55)
   <223> N = an equimolar mixture of A, C, G and T bases
<220>
   <221> misc_feature
   <222> (50)..(55)
   <223> N = an equimolar mixture of A, C, G and T bases of which the las t nucleotide is a 2'-3' dideoxynucleotide and the final phosphate bond has been exchanged with a phosphorothioate bond
<400> 1
   aattctaata cgactcacta tagggagaga aggataccac tagctagcgn nnnnn 55
<210> 2
   <211> 36
   <212> DNA
   <213> Artificial Sequence
<220>
   <223> Primer
<220>
   <221> misc_feature
   <222> (31) .. (36)
   <223> N represents a random hexamer -N stands for an equimolar mixture of A,C,G and T bases- of which the last nucleotide is a 2'-3' di deoxynucleotide
<400> 2
   aattctaata cgactcacta tagggagaga nnnnnn 36
<210> 3
   <211> 30
   <212> DNA
   <213> Artificial Sequence
<220>
   <223> Primer
<400> 3
   agcacggaaa gacgatgacg gaaaaagaga                                        30